(19) Europäisches Patentamt
European Patent Office
Office européen des brevets

(11) Veröffentlichungsnummer: **0 184 074 B1**

# (12) EUROPÄISCHE PATENTSCHRIFT

(45) Veröffentlichungstag der Patentschrift: **24.05.89**

(51) Int. Cl.[4]: **C07C 102/08**, C07C 103/365

(21) Anmeldenummer: **85114755.3**

(22) Anmeldetag: **21.11.85**

## (54) Verfahren zur Herstellung von N-Vinylformamid.

(30) Priorität: **29.11.84 DE 3443463**

(43) Veröffentlichungstag der Anmeldung:
**11.06.86 Patentblatt 86/24**

(45) Bekanntmachung des Hinweises auf die Patenterteilung:
**24.05.89 Patentblatt 89/21**

(84) Benannte Vertragsstaaten:
**AT BE CH DE FR GB IT LI NL SE**

(56) Entgegenhaltungen:
**BE-A- 677 368**
**DE-A- 1 950 280**
**DE-B- 1 014 094**

(73) Patentinhaber: **BASF Aktiengesellschaft, Carl-Bosch-Strasse 38, D-6700 Ludwigshafen(DE)**

(72) Erfinder: **Brunnmueller, Fritz, Dr., Alsenzstrasse 7, D-6703 Limburgerhof(DE)**
Erfinder: **Kroener, Michael, Dr., Eislebener Weg 8, D-6800 Mannheim 31(DE)**
Erfinder: **Goetze, Walter, Dr., Duerkheimer Strasse 13, D-6701 Dannstadt-Schauernheim(DE)**

**EP 0 184 074 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der DE-PS 1224304 ist unter anderem ein Verfahren zur Herstellung von N-Vinylformamid der Formel
$CH_2$=CH-NH-CHO (I)
bekannt, bei dem man Formylalaninnitril der Formel

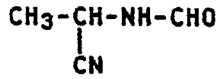
(II)

in Gegenwart von Feststoffen als Katalysator unter vermindertem Druck bei Temperaturen von vorzugsweise 450 bis 650°C pyrolisiert. Bei der Pyrolyse wird aus II unter Bildung von I Cyanwasserstoff abgespalten. Die Pyrolyseprodukte, sowie nicht-umgesetztes Formylalaninnitril, werden unter vermindertem Druck kondensiert und nach dem Druckausgleich mit der Atmosphäre einer fraktionierten Destillation unterworfen. Der Cyanwasserstoff, der bei der fraktionierten Destillation als erste Fraktion erhalten wird, wird zur Herstellung von neuem Ausgangsmaterial für die Pyrolyse verwendet. Bei einer solchen Verfahrensweise, die auch kontinuierlich durchgeführt werden kann, fallen jedoch erhebliche Mengen an Cyanwasserstoff an, für dessen Lagerung und Handhabung strenge Sicherheitsmaßstäbe anzuwenden sind.

Formylalaninnitril (II) kann beispielsweise durch Umsetzung von α-Amino-propionitril mit Ameisensäuremethylester oder nach dem Verfahren der US-PS 3822306 durch Reaktion von Acetaldehydcyanhydrin mit Formamid in Gegenwart von Säuren als Katalysator hergestellt werden.

Aufgabe der Erfindung ist es, ein Verfahren zur Herstellung von N-Vinylformamid zur Verfügung zu stellen, bei dem das Problem der Lagerung und Handhabung von größeren Mengen an Cyanwasserstoff nicht auftritt.

Die Aufgabe wird erfindungsgemäß mit einem Verfahren zur Herstellung von N-Vinylformamid der Formel
$CH_2$=CH-NH-CHO (I)
durch Pyrolyse von Formylalaninnitril der Formel

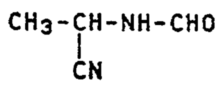
(II)

in Gegenwart von Feststoffen als Katalysator unter vermindertem Druck, Temperaturen von 250 bis 650°C und Abkühlen der Pyrolyseprodukte, die im wesentlichen aus (I) und Cyanwasserstoff bestehen, ebenfalls unter vermindertem Druck und Isolierung von (I) dadurch gelöst, daß man die Pyrolyseprodukte unter einem Druck von 10 bis 150 mbar auf eine Temperatur von 200 bis -10°C abkühlt, so daß eine weitgehende fraktionierte Trennung in kondensiertes (I) und gasförmigen Cyanwasserstoff erfolgt, (I) aus dem Kondensat isoliert und den nicht abgetrennten gasförmigen Cyanwasserstoff anschließend unter einem Druck von 5 bis 140 mbar in etwa molarem Verhältnis einer Chemiesorption mit Acetaldehyd bei Temperaturen von -20 bis +30°C unter Bildung von Milchsäurenitril unterwirft und es nach dem Druckausgleich mit der Atmosphäre isoliert.

Die Pyrolyse von (II) erfolgt an Feststoffen. Man kann hierfür diejenigen Feststoffe verwenden, die in der DE-PS 1224304 angegeben sind, sowie Alkali- und Erdalkalicarbonate, wie Natriumcarbonat, Kaliumcarbonat, Magnesiumcarbonat, Strontiumcarbonat, Bariumcarbonat, Marmor, Dolomit, Kreide und Magnesit. Außerdem eignen sich auch Magnesiumoxid, Kalziumoxid und Bariumoxid. Als besonders vorteilhaft haben sich solche Katalysatoren erwiesen, die Alkali- und/oder Erdalkalicarbonate auf α-Aluminiumoxid als Träger enthalten. Vorzugsweise verwendet man Kalziumcarbonat, Dolomit oder Mischungen aus Kalziumcarbonat und Magnesiumcarbonat auf α-Aluminiumoxid. Katalysatoren dieser Art werden hergestellt, indem man α-Aluminiumoxid mit wasserlöslichen Salzen, z.B. Calciumacetat, tränkt und durch eine thermische Behandlung in die entsprechenden Carbonate bzw. Oxide überführt.

Die Pyrolyse wird bei Temperaturen von 250 bis 650, vorzugsweise 300 bis 550°C und unter einem Druck von 10 bis 150, vorzugsweise 10 bis 50 mbar durchgeführt. Bei der Pyrolyse wird aus der Verbindung der Formel (II) unter Bildung von N-Vinylformamid (I) Cyanwasserstoff abgespalten. Die Pyrolyseprodukte bestehen im wesentlichen aus (I) und Cyanwasserstoff, enthalten jedoch noch geringe Mengen an nicht umgesetztem (II) sowie geringe Mengen an Verunreinigungen.

Es hat sich als besonders vorteilhaft erwiesen, die Pyrolyse von (II) in Gegenwart von 0,1 bis 2 Mol Formamid, bezogen auf ein Mol der Verbindung der Formel (II) durchzuführen. Formamid erfüllt hierbei mehrere Funktionen. Es wirkt als Verdünnungsmittel bei der Pyrolyse und führt hier zu einer Verlängerung der Standzeiten der Katalysatoren. Außerdem wird die Gewinnung von besonders reinem N-Vinylformamid durch fraktionierte Destillation in Gegenwart von Formamid erleichtert.

In Abhängigkeit von der Auswahl des Katalysators wird Formamid bei der Pyrolyse teilweise in Cyanwasserstoff und Wasser gespalten. Die zusätzliche Bildung von Cyanwasserstoff aus Formamid ist erwünscht, um in einem Recycling-Prozess eine ausreichende Menge an Cyanwasserstoff für die Herstellung des Ausgangsprodukts (II) zur Verfügung zu haben. Verwendet man bei der Pyrolyse verschiedene Katalysatoren, so kann man die Reaktion in der Weise steuern, daß zunächst die Verbindung der Formel (II) bevorzugt pyrolisiert wird und das Reaktionsgemisch dann über einen Kontakt leiten, der bevorzugt Formamid abspaltet. Kontakte, die bevorzugt Formamid spalten, sind beispielsweise aus der DE-PS 1209561 bekannt. Es handelt sich hierbei hauptsächlich um Eisen-Zink-Oxide. Die Pyrolyse kann durch unterschiedliche Schüttungen der verschiedenen Katalysatortypen sowie außerdem durch unterschiedliche Temperaturprofile im Pyrolysereaktor gesteuert werden.

Die Reaktionsprodukte, die den Pyrolysereaktor verlassen, werden in dem Druckbereich von 10 bis 150 mbar auf eine Temperatur in dem Bereich von 200 bis -10, vorzugsweise 60 bis -10°C abgekühlt, so daß eine weitgehende fraktionierte Trennung in kondensiertes N-Vinylformamid und gasförmigen Cyanwasserstoff erfolgt. Sofern die Pyrolyse in Gegenwart von Formamid durchgeführt wird, kondensiert man das nicht gespaltene Formamid zusammen mit N-Vinylformamid und gegebenenfalls unverändertem (II) aus. Wird außerdem nicht das gesamte Formylalaninnitril (II) pyrolysiert, so enthält das kondensierte N-Vinylformamid das nicht umgesetzte (II). Es bereitet keinerlei Schwierigkeiten, N-Vinylformamid von (II) bzw. Formamid destillativ voneinander zu trennen. Das Abkühlen der Pyrolysegase wird vorzugsweise in zwei oder mehreren voneinander getrennten Kühlern, die jeweils über Abscheider verfügen, vorgenommen. Beispielsweise kühlt man die Pyrolysegase in einem ersten Wärmetauscher auf eine Temperatur von 60°C in einem zweiten auf 20°C und in einem folgenden dritten Kühler auf eine Temperatur von -10°C, wobei die aus den einzelnen Wärmetauschern stammenden Kondensate separat gesammelt werden. Man erreicht so eine fraktionierte Trennung des Pyrolysegases in die wesentlichen Bestandteile, nämlich kondensiertes N-Vinylformamid und gasförmigen Cyanwasserstoff. Ein Ausfrieren von Cyanwasserstoff, das bei den bekannten Verfahren praktiziert wird, entfällt bei dem erfindungsgemäßen Verfahren.

Aus den Kondensaten kann N-Vinylformamid durch fraktionierte Destillation gewonnen werden, wobei geringe Mengen an Blausäure, die in dem kondensierten N-Vinylformamid gelöst sind, wiederverwendet werden können.

Der aus der Pyrolyse stammende Cyanwasserstoff wird nach der Kondensation der hochsiedenden Bestandteile unter einem Druck von 5 bis 140, vorzugsweise 5 bis 45 mbar in etwa molarem Verhältnis einer Chemiesorption mit Acetaldehyd bei Temperaturen von -20 bis +30, vorzugsweise -5 bis +15°C unterworfen. Hierbei bildet sich Milchsäurenitril, das in Formamid als Lösungsmittel aufgenommen werden kann. Man setzt entweder nach der Chemiesorption Formamid zu oder nimmt vorzugsweise die Chemiesorption des Cyanwasserstoffs in Gegenwart von Formamid als Lösungsmittel vor. Durch Zugabe geringer Mengen einer Base, vorzugsweise tert. Aminen, wie Triethylamin, wird die Basizität der Chemiesorptionslösung so gesteuert, daß die Reaktion zwischen Acetaldehyd und Cyanwasserstoff außerordentlich schnell abläuft. Die Menge an Base beträgt 0,005 bis 0,02 Mol, bezogen auf ein Mol Cyanwasserstoff.

Es ist als überraschend anzusehen, daß die Reaktionspartner mit so hohen Dampfdrücken wie Cyanwasserstoff (Kp 27°C bei Normaldruck) und Acetaldehyd (Kp 21°C bei Normaldruck) unter vermindertem Druck bis zu 5 mbar praktisch quantitativ zur Reaktion gebracht werden können. Die Regelung der Acetaldehydzugabe kann über das Potential einer Silber-Elektrode erfolgen und wird so eingestellt, daß sich eine Cyanwasserstoffkonzentration in der Formamid-Lösung von vorzugsweise zwischen 0,05 und 0,1 Gew.% einstellt. Die Aminzugabe kann über das Potential einer Glaselektrode gesteuert werden. Es ist jedoch auch eine Vakuum-gesteuerte Aminzugabe möglich, weil bei zu geringer Konzentration an tert. Amin in der Formamidlösung die Reaktionsgeschwindigkeit der Blausäure mit Acetaldehyd abnimmt und es dadurch zu einer Erhöhung des Drucks käme, so daß das Vakuum zusammenbrechen könnte.

Die Chemiesorption des Cyanwasserstoffs kann beispielsweise in einer Kolonne durchgeführt werden, die je nach Bedarf kühlbar bzw. beheizbar ist. Vorzugsweise versprüht man am Kopf der Kolonne eine Lösung von Formamid, Acetaldehyd und einer tertiären Base und leitet den Cyanwasserstoff ebenfalls an dieser Stelle der Kolonne ein. In einem Abscheider sammelt sich dann eine Lösung von Milchsäurenitril in Formamid, aus der Milchsäurenitril nach dem Druckausgleich mit der Atmosphäre isoliert werden kann. Bei kontinuierlichem Betrieb führt man eine Milchsäurenitril enthaltende Formamidlösung im Kreis, in den man vor der Chemiesorption des Cyanwasserstoffs Formamid, Acetaldehyd und tert. Amin einspeist und nach erfolgter Chemiesorption Milchsäurenitril und Formamid ausschleust. In dem Chemiesorptionskreislauf wird vorzugsweise mit einer solchen Menge an Formamid gearbeitet, daß man eine Lösung von Milchsäurenitril in Formamid im Molverhältnis von 1:1,5 bis 2, vorzugsweise 1:1,8 erhält. Diese Lösung kann nach dem Verfahren der US-PS 3822306 zu dem Ausgangsprodukt (II) kondensiert werden, das dann der Pyrolyse zur Herstellung von (I) unterworfen werden kann.

Ein besonders interessanter Aspekt dieser Verfahrensweise ist in der Tatsache zu sehen, daß die in einer technischen Anlage stündlich anfallenden Cyanwasserstoffmengen - bei einem Einsatz von 10 kMol/Stunde sind dies 270 kg Cyanwasserstoff - in Bruchteilen von Sekunden abreagieren und eine unmittelbar zurückführbare Lösung von Milchsäurenitril in Formamid mit einem Gehalt von höchstens 0,1 Gew.% Cyanwasserstoff liefern. Das erfindungsgemäße Verfahren bedeutet somit einen außerordentlichen Fortschritt für die sicherheitstechnischen Belange.

Beispiel 1

Fraktionierte Trennung von N-Vinylformamid und Cyanwasserstoff

Der Pyrolysereaktor besteht aus einem senkrecht stehenden Edelstahlrohr mit einem Durchmesser von 40 mm und einer Länge von 650 mm. Der Reaktor ist mit 3 voneinander unabhängigen Heizzonen beheizbar. Dem Reaktor ist ein Dünnschichtverdampfer vorgeschaltet, der Reaktorausgang ist mit drei Kondensatoren verbunden, in denen die Spaltgase stufenweise abgekühlt werden, und zwar im 1. Kondensator auf 50°C, im 2. auf -5°C und im 3. auf -80°C. (Das fraktionierte Kühlen der Spaltgase auf 50 und -5°C ist ein wesentliches Merkmal der vorliegenden Erfindung.) Das Edelstahlrohr wird für die Versuche a) bis d) jeweils mit den in Tabelle 1 angegebenen Katalysatoren gefüllt. Die Apparatur wird dann so evakuiert, daß im Dünnschichtverdampfer ein Druck von 30 mbar aufrechterhalten wird. Man leitet 10 Nl Kohlenmonoxid pro Stunde durch die Apparatur und heizt den Katalysator auf eine Temperatur von 380 bis 420°C auf.

Über einen Zeitraum von 12 Stunden werden dann stündlich 331 g (3,38 Mol) der Verbindung (II) und 134 g (2,98 Mol) Formamid verdampft. Die Dämpfe passieren die Katalysatorschichten in dem oben angegebenen Temperaturbereich und werden dann in den Kondensatoren auf 50,-5 bzw. -80°C kondensiert. Die Fraktionen 1 und 2 enthalten N-Vinylformamid, während das Kondensat 3 (erhalten durch Abkühlung auf -80°C) aus fast reinem Cyanwasserstoff besteht. Die Umsätze an Verbindung der Formel (II) und Formamid bzw. Ausbeuten an Formamid und Blausäure sind in der Tabelle angegeben.

Tabelle

| Beispiel 1 | Katalysator* | Belastung g/lh | Reaktorinnentemperatur | | | Umsätze (%) | | Ausbeuten (%) | |
|---|---|---|---|---|---|---|---|---|---|
| | | | oben | Mitte | unten | Verb. II | Formamid | I (bez. auf II) | HCN (bez. auf II + Formamid |
| a) | A | 580 | 385 | 420 | 420 | 96 | 2 | 88 | 100 |
| b) | oben: 40 cm A<br>unten: 25 cm B | 614 | 385 | 420 | 420 | 95 | 20 | 86 | 100 |
| c) | oben: 40 cm A<br>unten: 25 cm C | 609 | 380 | 420 | 380 | 92 | 20 | 86 | 98 |
| d) | D | 680 | 400 | 420 | 420 | 93 | 25 | 88 | 100 |

*Katalysator:
A: $CaCO_3$ auf MgO, 8–14 mm Splitt
B: $MgCO_3$ auf $\alpha$-$Al_2O_3$, 8–14 mm Splitt
C: $MgCO_3$ auf $\alpha$-$Al_2O_3$, 14–20 mm Splitt
D: $CaCO_3$ auf $\alpha$-$Al_2O_3$, 8–1 mm Splitt
E: Ca/$MgCO_3$ auf $\alpha$-$Al_2O_3$

Beispiel 2

Die Pyrolyse wird in einem Rohr aus Edelstahl durchgeführt, das einen Durchmesser von 53 mm und eine Länge von 1000 m hat und mit dem in Tabelle 1 beschriebenen Katalysator E gefüllt ist. Dem Pyrolyserohr ist ein Dünnschichtverdampfer vorgeschaltet. Die Pyrolysegase werden in zwei Kondensatoren abgekühlt, und zwar im 1. Kondensator auf eine Temperatur von 50°C und im 2. auf eine Temperatur von –5°C. Die nichtkondensierten Bestandteile, in der Hauptsache Cyanwasserstoff, werden in einen Chemiesorptions-Kreisreaktor geleitet. Diese Vorrichtung besteht aus einer Füllkörperkolonne, an die sich ein Abscheider anschließt. Ca. 5 kg Formamid werden über eine Düse am Kopf der Kolonne, die mit der Pyrolyseapparatur verbunden ist, im Kreis gepumpt. Um einen stationären Zustand zu erreichen, wird in den im Kreislauf geführten Formamidstrom zusätzlich Formamid, Acetaldehyd und Triethylamin als Katalysator eingeführt. In der Füllkörperkolonne vollzieht sich dann die Chemiesorption des Cyanwasserstoffs. Inerte Gase, die als Nebenprodukte entstehen, werden am Kopf des Abscheiders über eine Vakuumpumpe abgezogen. Es wird ein Druck von 20 mbar aufrechterhalten. Die Temperatur der im

Kreis geführten Chemiesorptionslösung beträgt 5°C. In den Kreislauf ist außerdem zwischen Abscheider und Umwälzpumpe ein Kühler eingebaut, mit dem die im Kreis geführte Formamidlösung auf eine Temperatur von 5°C gehalten wird.

Die oben beschriebene Apparatur wird kontinuierlich betrieben, indem man stündlich 1478 g Formylalaninnitril (II) im Dünnschichtverdampfer verdampft und über den Katalysator E leitet, dessen oberes Drittel auf eine Temperatur von 400 und der Rest auf eine Temperatur von 420°C erhitzt ist. In den beiden Kondensatoren scheiden sich stündlich neben 74 g nicht umgesetztem (II) und wenigen Nebenprodukten, wie Formamid, Cyanwasserstoff und Milchsäurenitril, 895 g N-Vinylformamid ab, das aus den Kondensatoren entnommen und einer fraktionierten Destillation zugeführt wird. Die Ausbeute an N-Vinylformamid beträgt 88 %, bezogen auf umgesetztes (II).

Der nicht-kondensierte Cyanwasserstoff wird am Kopf der Füllkörperkolonne mit einer Formamidlösung in Kontakt gebracht, die über eine Düse in die Füllkörperkolonne eingesprüht wird und in die vor der Düse jeweils stündlich 594 g Acetaldehyd, 1094 g Formamid und 10 g Triethylamin kontinuierlich eindosiert werden. Gleichzeitig entnimmt man stündlich dem Kreis am Boden des Abscheiders 2063 g einer Reaktionslösung, die nach Erreichen des stationären Zustandes 46,2 Gew.% Milchsäurenitril, in Formamid gelöst, neben sehr geringen Mengen an Triethylamin und Wasser enthält. Die aus dem Kreis abgezogene Lösung von Milchsäurenitril in Formamid enthält weniger als 0.1 Gew.% freie Blausäure. Sie wird zur Stabilisierung mit Ameisensäure versetzt und kann zu II kondensiert und anschließend erfindungsgemäß pyrolisiert werden.

**Patentansprüche**

1. Verfahren zur Herstellung von N-Vinylformamid der Formel
$CH_2$=CH–NH–CHO (I)
durch Pyrolyse von Formylalaninnitril der Formel

$CH_3$–CH–NH–CO (II)
|
CN

in Gegenwart von Feststoffen als Katalysator unter vermindertem Druck, Temperaturen von 250 bis 650°C und Abkühlen der Pyrolyseprodukte, die im wesentlichen aus I und Cyanwasserstoff bestehen, ebenfalls unter vermindertem Druck und Isolierung von I, dadurch gekennzeichnet, daß man die Pyrolyseprodukte unter einem Druck von 10 bis 150 mbar auf eine Temperatur von 200 bis –10°C abkühlt, daß eine weitgehende fraktionierte Trennung in kondensiertes I und gasförmigen Cyanwasserstoff erfolgt, I aus dem Kondensat isoliert und den nicht abgetrennten gasförmigen Cyanwasserstoff anschließend unter einem Druck von 5 bis 140 mbar in etwa molarem Verhältnis einer Chemiesorption mit Acetaldehyd bei Temperaturen von –20 bis +30°C unter Bildung von Milchsäurenitril unterwirft und es nach dem Druckausgleich mit der Atmosphäre isoliert.

2. Verfahren nach Anspruch 1, dadurch gekennzeichnet, daß man die Pyrolyse unter einem Druck von 10 bis 50 mbar und einer Temperatur von 300 bis 550°C und die Chemiesorption des Cyanwasserstoffs unter einem Druck von 5 bis 45 mbar und einer Temperatur von -5 bis +15°C in Gegenwart katalytisch wirkender Mengen einer Base durchführt.

3. Verfahren nach den Ansprüchen 1 und 2, dadurch gekennzeichnet, daß man die Pyrolyse von II in Gegenwart von 0,1 bis 2 Mol Formamid, bezogen auf 1 Mol II, durchführt.

4. Verfahren nach den Ansprüchen 1 bis 3, dadurch gekennzeichnet, daß man als Träger für die Katalysatoren $\alpha$-Aluminiumoxid verwendet.

5. Verfahren nach den Ansprüchen 1 bis 4, dadurch gekennzeichnet, daß man die Chemiesorption des Cyanwasserstoffs in Gegenwart von Formamid vornimmt, wobei man anschließend die dabei entstehende Mischung aus Milchsäurenitril und Formamid in bekannter Weise in Gegenwart von Säuren als Katalysator zu II umsetzt.

6. Verfahren nach den Ansprüchen 1 bis 5, dadurch gekennzeichnet, daß man die Reaktionen jeweils kontinuierlich durchführt.

7. Verfahren nach Anspruch 2, dadurch gekennzeichnet, daß man nach der Chemiesorption des Cyanwasserstoffs Formamid zusetzt.

8. Verfahren nach Anspruch 5, dadurch gekennzeichnet, daß man die Pyrolyse von II, die fraktionierte Trennung von I und Cyanwasserstoff, die Chemiesorption des Cyanwa sserstoffs, die Umsetzung des Milchsäurenitrils und Formamids zu II kontinuierlich durchführt und das so erhaltene II bei der Pyrolyse einsetzt.

## Claims

1. A process for the preparation of N-vinylformamide of the formula
$CH_2$=CH–NH–CHO (I)
by pyrolysis of formylalaninenitrile of the formula

$CH_3$-CH-NH-CHO (II)
|
CN

in the presence of a solid as a catalyst under reduced pressure, at from 250 to 650°C and with cooling of the pyrolysis products, which essentially consist of I and hydrogen cyanide, likewise under reduced pressure and with isolation of I, wherein the pyrolysis products are cooled to a temperature of from 200 to –10°C under from 10 to 150 mbar so that substantial fractionation into condensed I and gaseous hydrogen cyanide takes place, I is isolated from the condensate, and the gaseous hydrogen cyanide which has not been separated off is then subjected to chemisorption with about an equimolar amount of acetaldehyde at from –20 to +30°C under from 5 to 140 mbar with formation of lactonitrile, and the latter is isolated after the pressure has been brought to atmospheric pressure.

2. A process as claimed in claim 1, wherein the pyrolysis is carried out under from 10 to 50 mbar and at from 300 to 550°C, and the chemisorption of the hydrogen cyanide is effected under from 5 to 45 mbar and at from –5 to +15°C in the presence of a catalytic amount of a base.

3. A process as claimed in either of claims 1 and 2, wherein the pyrolysis of II is carried out in the presence of from 0.1 to 2 moles of formamide per mole of II.

4. A process as claimed in any of claims 1 to 3, wherein α-alumina is used as a carrier for the catalysts.

5. A process as claimed in any of claims 1 to 4, wherein the chemisorption of the hydrogen cyanide is carried out in the presence of formamide, the resulting mixture of lactonitrile and formamide subsequently being reacted in a conventional manner, in the presence of an acid as a catalyst, to give II.

6. A process as claimed in any of claims 1 to 5, wherein each of the reactions is carried out continuously.

7. A process as claimed in claim 2, wherein formamide is added after the chemisorption of the hydrogen cyanide.

8. A process as claimed in claim 5, wherein the pyrolysis of II, the fractionation of I and hydrogen cyanide, the chemisorption of the hydrogen cyanide and the reaction of lactonitrile and formamide to give II are carried out continously, and the II thus obtained is used for the pyrolysis.

## Revendications

1. Procédé de préparation de N-vinylformamide de formule
$CH_2$=CH–NH–CHO (I)
par pyrolyse de formylalaninenitrile de formule

$CH_3$–CH–NH–CO (II)
|
CN

en présence de matières solides en tant que catalyseur dans des conditions de pression réduite et de température comprise entre 250 et 650°C, et refroidissement des produits de pyrolyse, qui se composent du composé de formule I et d'acide cyanhydrique, également sous pression réduite, et isolement du composé I, caractérisé en ce que l'on refroidit les produits de pyrolyse sous une pression de 10 à 150 mbar d'une température de 200° à une température de –10°C, ce qui provoque une séparation fractionnée importante du produit I condensé et de l'acide cyanhydrique gazeux, on isole le produit I du condensat et on soumet ensuite l'acide cyanhydrique gazeux non séparé à une chimisorption, sous une pression de 5 à 140 mbar et une température de –20 à +30°C, avec de l'acétaldéhyde en proportion à peu près molaire avec formation de lactonitrile et on l'isole par égalisation de la pression avec la pression atmosphérique.

2. Procédé selon la revendication 1, caractérisé en ce qu'on effectue la pyrolyse sous une pression de 10 à 50 mbar et à une température de 300° à 550°C, et la chimisorption de l'acide cyanhydrique sous une pression de 5 à 45 mbar et à une température de –5 à +15°C en présence d'une quantité catalytiquement active d'une base.

3. Procédé selon la revendication 1 ou 2, caractérisé en ce qu'on effectue la pyrolyse du composé II en présence de 0,1 à 2 moles de formamide pour 1 mole du composé II.

4. Procédé selon l'une quelconque des revendications 1 à 3, caractérisé en ce qu'on utilise de l'oxyde d'aluminium α en tant que support du catalyseur.

5. Procédé selon l'une quelconque des revendications 1 à 4, caractérisé en ce qu'on effectue la chimisorption de l'acide cyanhydrique en présence de formamide, grâce à quoi on transforme immédiatement en composé II le mélange ainsi obtenu de lactonitrile et de formamide de façon connue en présence d'acides en tant que catalyseur.

6. Procédé selon l'une quelconque des revendications 1 à 5, caractérisé en ce qu'on effectue les réactions éventuellement en continu.

7. Procédé selon la revendication 2, caractérisé en ce qu'on ajoute le formamide après la chimisorption de l'acide cyanhydrique.

8. Procédé selon la revendication 5, caractérisé en ce qu'on effectue en continu la pyrolyse du composé II, la séparation fractionnée du composé I et de l'acide cyanhydrique, la chimisorption de l'acide cyanhydrique, la transformation du lactonitrile et du formamide en composé II et on introduit le composé II ainsi obtenu à l'étape de pyrolyse.